# FASCICULE DE BREVET EUROPEEN

(11) **EP 0 312 413 B1**
(45) Date de publication et mention de la délivrance du brevet: **05.05.1993**
(21) Numéro de dépôt: 88402391.2
(22) Date de dépôt: 22.09.1988
(51) Int. Cl.: A61F 7/00, A61N 5/06

(54) **Appareil portatif pour le chauffage localisé de la peau à des fins thérapeutiques**
Tragbares Gerät zur lokalen Erwärmung der Haut für therapeutische Zwecke
Portable device for the local heating of the skin for therapeutic purposes

(30) Priorité: 07.10.1987 FR 8713825
(43) Date de publication de la demande: 19.04.1989
(73) Titulaire: Ratkoff, Georges, F-28120 Illiers Combray (FR); Deheuvels, Jean-Paul, F-28630 Chartres (FR)
(72) Inventeur: Ratkoff, Georges, F-28120 Illiers Combray (FR); Deheuvels, Jean-Paul, F-28630 Chartres (FR)
(74) Mandataire: Chevallier, Robert Marie Georges

(56) Documents cités:
- DE-A- 3 112 676
- DE-A- 3 301 802
- US-A- 2 906 264
- US-A- 4 261 364
- US-A- 4 658 823

## Description

L'invention a pour objet un appareil portatif pour le chauffage localisé de la peau destiné à un usage thérapeutique, notamment pour la suppression des effets désagréables et même douloureux provoqués par les piqûres d'insectes tels que les hyménoptères (abeilles, guêpes, frelons, fourmis, ...) et des animaux marins comme les vives, rascasses, méduses, etc...

On sait que le venin de ces animaux est détruit par la chaleur. Une méthode ancienne connue consiste à chauffer fortement la région atteinte par une piqûre en approchant de l'endroit piqué une cigarette allumée ou un objet chaud comme un fer chauffé au rouge.

Cette méthode est difficile à appliquer; elle ne permet pas de chauffer la peau sans risque de brûlure, de manière suffisamment précise, exactement à l'endroit voulu, à la température minimale nécessaire et pendant la durée suffisante pour garantir la destruction du venin.

Il n'existe aujourd'hui aucun appareil petit, léger, facilement emportable avec soi et aisément maniable pour effectuer rapidement et en toutes circonstances un chauffage localisé d'une région de la peau où une piqûre a été faîte par un insecte ou un animal marin à venin thermolabile.

L'invention a donc pour but principal d'apporter un tel appareil de chauffage ayant les qualités et les avantages mentionnés ci-dessus.

Bien que l'usage de l'appareil de l'invention est destiné au traitement des zones piquées, il est évidemment utilisable partout où il existe un besoin de chauffage localisé de la peau pour une raison quelconque.

On connait déjà des appareils conçus pour un chauffage localisé de la peau, par exemple par les documents FR-A-1.139.096, US-A-4.505.545 et US-A-4.658.823.

Les appareils décrits dans ces trois documents comprennent un corps creux allongé ayant une extrémité ouverte, une ampoule électrique montée à l'intérieur de ce corps à proximité de l'extrémité opposée qui est fermée, un réflecteur placé derrière cette ampoule du côté de l'extrémité fermée du corps. Ces appareils sont utilisés par la mise de cette extrémité fermée en contact avec la peau, ou à proximité de celle-ci, pendant que l'ampoule est alimentée en courant électrique et sert de source de chaleur.

Mais ces trois appareils sont conçus pour être alimentés directement par le réseau électrique; la puissance des ampoules quand elle est mentionnée est de 15 à 25 W au minimum et de plusieurs centaines de Watts au maximum. Ces appareils ne peuvent donc pas être emportés avec soi et ne peuvent pas être alimentés par des piles ou des accumulateurs suffisamment petits pour pouvoir être facilement transportables. En outre, le flux calorifique qu'ils produisent n'est pas déterminé avec précision. Leur but est de produire un certain échauffement de la peau jusqu'à une température qui n'est pas indiquée. L'appareil du document US-A-4.658.823 déjà cité comprend un thermostat mais celui-ci n'est prévu que comme un moyen de coupure quand une valeur limite supérieure de la température, pouvant être dangereuse pour l'utilisateur, risque d'être atteinte; cet appareil est destiné à chauffer l'ensemble d'une oreille et il comprend aussi des ouvertures à section réglable d'entrée d'air extérieur froid sans contrôle de la température.

Or les venins sensibles à la chaleur sont détruits selon les cas à une température comprise entre 50°C et 60°C qui doit leur être appliquée pendant une durée comprise entre 20 à 30 secondes pour les hyménoptères et 1 à 3 minutes pour les animaux marins. De plus, comme le venin commence à se diffuser dans les tissus dès l'instant de la piqûre il est souhaitable que la température efficace soit appliquée à la peau sans perte de temps. En pratique, dès que l'appareil mis en place contre la peau commence à échauffer celle-ci, il est souhaitable que la température efficace de 50° à 60°C soit atteinte en moins d'une minute.

Si l'on tient compte que la valeur de 60°C est le début de la zone de température que la peau peut supporter pendant quelques instants avant qu'une brûlure commence à se produire, il est tout à fait clair que les appareils connus n'ont pas les caractéristiques qui leur permettraient de traiter à coup sûr les piqûres, sans brûler la peau.

On remarquera que l'emploi d'un thermostat, qui peut éliminer le risque de brûlure, a pour inconvénient d'augmenter le poids et d'accroître le prix de l'appareil. Or, pour être portatif et d'usage courant, l'appareil doit, en plus, être aussi léger et aussi économique que possible.

Un appareil qui convient pour le chaufface localisé pour la destruction du venin injecté dans la peau par la piqûre de certains insectes et animaux marins est décrit dans la revendication 1.

Quand on utilise l'appareil de l'invention, on applique l'extrémité ouverte de l'appareil contre la peau, de sorte que la puissance par unité de surface mentionnée ci-dessus est celle qui est réalisée sur la peau dans une zone qui contient l'endroit exact de la piqûre, celle-ci devant être de préférence au centre de cette zone.

Cette densité de la puissance peut être obtenue à l'aide de nombreuses sources différentes de flux calorifique; dans le cadre de l'invention cette source doit avoir une puissance comprise entre 0,8 W et 3 W.

Comme on le montrera plus loin, une résistance électrique, alimentée par une pile électrique ou un accumulateur électrique servant de source d'énergie, peut convenir. Cependant il est préférable d'adopter comme source de flux électrique une ampoule électrique développant une puissance comprise entre 0,12 W/mm² à 0,20 W/mm², la surface étant celle de la section de cette ampoule par un plan parallèle au plan contenant l'extrémité ouverte du corps de l'appareil, et la distance entre ce plan contenant l'extrémité ouverte et le point de l'ampoule le plus proche de ce même plan étant de 2 mm environ.

Selon un mode de réalisation préféré, l'ampoule électrique utilisée a une puissance de 1, 25 W environ et un diamètre extérieur mesuré dans un plan parallèle au plan de l'extrémité ouverte de 3 mm environ de sorte qu'elle développe une puissance de 0,17 W/mm²; la surface libre de l'extrémité ouverte est de 28 mm² environ et elle est située à une distance de 2 mm environ du point de l'ampoule le plus proche; avantageusement l'ampoule électrique a une tension nominale de 5 V environ et la source d'énergie qui l'alimente a une tension nominale de 9 V environ.

Pour bien faire comprendre l'invention, on donnera maintenant une description de plusieurs modes de réalisation d'un appareil portatif pour chauffage localisé de la peau en vue de la destruction de venin injecté par une piqûre d'insecte ou d'animal marin. On se reportera aux dessins annexés dans lesquels :
- La figure 1 est une vue partielle en coupe d'un appareil selon l'invention en cours d'utilisation sur la peau à l'endroit d'une piqûre,
- la figure 2 est une vue schématique en coupe de l'appareil complet de la figure 1,
- la figure 3 est une vue en coupe par un plan longitudinal d'un exemple préféré de réalisation selon l'invention,
- la figure 4 est une vue en coupe par un plan longitudinal de l'appareil de la figure 3, le plan de coupe étant perpendiculaire au plan de coupe de la figure 3,
- la figure 5 est une vue agrandie de détail montrant le montage de la source de flux calorifique de cet appareil.

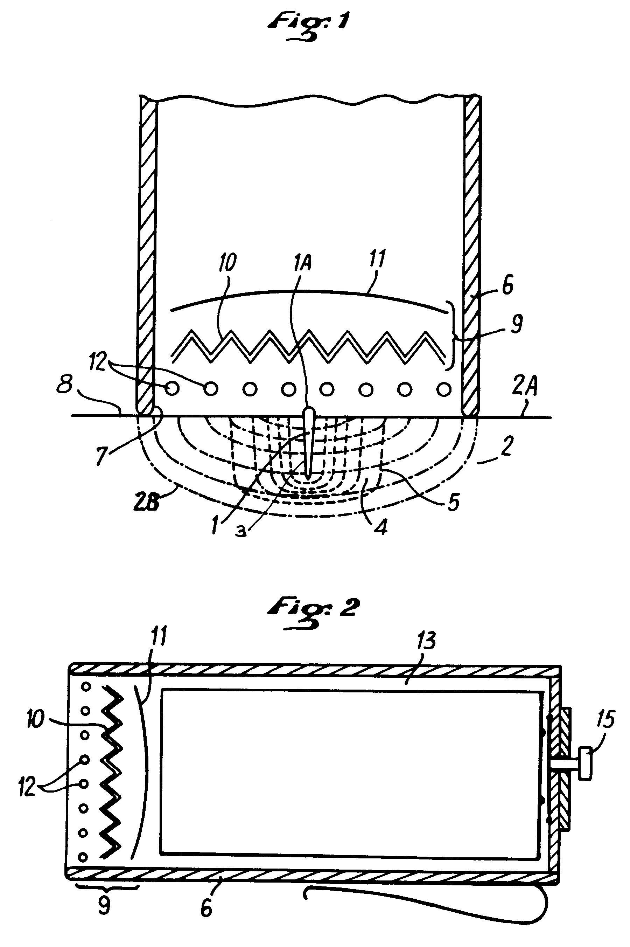

La figure 1 sert aussi à expliquer l'effet d'une piqûre,d'abeille par exemple, quand un dard 1 a pénétré dans la peau 2 d'un sujet. En général, le dard 1 est enduit sur sa partie externe 3 d'un venin qui se répand par diffusion dans la région 4 de la peau 2, au voisinage de l'endroit piqué. La diffusion du venin est représentée par un jeu de lignes 5 en trait interrompu qui s'éloignent progressivement du dard 1. Ce dernier a souvent une partie 1A qui dépasse de la surface 2A de la peau 2.

La figure 1 montre en coupe une partie d'un appareil conforme à l'invention appliqué contre la peau 2, autour du dard 1 ou de l'endroit de la piqûre. Cet appareil a un corps creux allongé 6 avec une extrémité ouverte 7 contenue dans un plan 8. Un élément chauffant 9 est fixé à l'intérieur du corps 6 de façon à être légèrement en retrait par rapport au plan 8 de l'extrémité ouverte 7.

Cet élément chauffant 9 est capable d'émettre un flux calorifique, vers l'extérieur du corps 6 par l'extrémité ouverte 7. Il est constitué avantageusement par une résistance électrique à laquelle est associé un réflecteur 11 placé dans le corps 6 en arrière de cette résistance par rapport à l'extrémité ouverte 7.

La position en retrait de l'élément chauffant 9 par rapport au plan 8 de l'extrémité ouverte 7 a pour but à la fois d'éviter d'enfoncer plus le dard 1 dans la peau, quand il est resté accroché à celle-ci, d'autre part de rendre impossible le contact direct entre cet élément chauffant 9 et la peau. Une distance de 2 mm environ entre le plan 8 de l'extrémité ouverte 7 et le point de l'élément chauffant 9 le plus proche de ce plan est convenable quand le rapport entre la puissance de cet élément chauffant 9 et la surface de l'extrémité ouverte 7 est compris entre 0,03 W/mm² et 0,30 W/mm², comme on l'a indiqué plus haut. La différence entre ces deux valeurs extrêmes se traduit essentiellement par une différence de rapidité d'échauffement de la peau. Il est préférable cependant d'adopter un rapport compris entre 0,04 W/mm² et 0,25 W/mm².

On utilise l'appareil de l'invention en appliquant contre la surface 2A de la peau 2 le plan 8 contenant l'extrémité ouverte 7 et en mettant en action l'élément chauffant 9. Le rayonnement chauffant émis par ce dernier traverse le volume d'air qui existe entre ce même élément 9 et la surface 2A de la peau 2, pénètre dans celle-ci progressivement, comme on l'a indiqué sur la figure 1 par un jeu de lignes 2B en trait mixte. La chaleur pénètre dans la peau et intéresse tout le volume où se trouve le venin. Ce dernier est détruit quand ce volume est porté à la température indiquée plus haut pendant la durée spécifiée.

En pratique, l'extrémité ouverte 7 est avantageusement circulaire, elle ne doit pas être trop faible en diamètre pour que le dard 1 puisse être entouré sans difficulté; elle ne doit pas être trop grande en diamètre pour que le volume à chauffer ne soit pas excessif. Il a été constaté que le choix d'un diamètre de 6mm, c'est-à-dire d'une surface de 28 mm² de l'extrémité ouverte 7 dans le plan 8 est une valeur optimale. Une telle surface permet de parvenir à une valeur du rapport défini plus haut avec un élément chauffant ayant une puissance assez modérée de 1,25 W environ permettant la réalisation d'un appareil portatif autonome, à durée d'utilisation suffisamment longue pour être acceptable en pratique.

Il est possible, si on le juge souhaitable, de placer une grille 12 de protection, dans le corps 6, entre le plan 8 de l'extrémité ouverte 7 et l'élément chauffant 9. Cette grille assure à la fois la protection mécanique de l'élément chauffant et elle interdit positivement tout contact direct avec ce dernier. Selon une variante (non représentée) la grille 12 peut être remplacée par une paroi pleine transparente au flux calorifique émis par l'élément chauffant.

Quand l'appareil satisfait aux conditions générales mentionnées plus haut, il assure efficacement son rôle mais il est compréhensible que pour parvenir à l'appareil le plus économique possible la réalisation de l'élément chauffant a la plus grande importance.

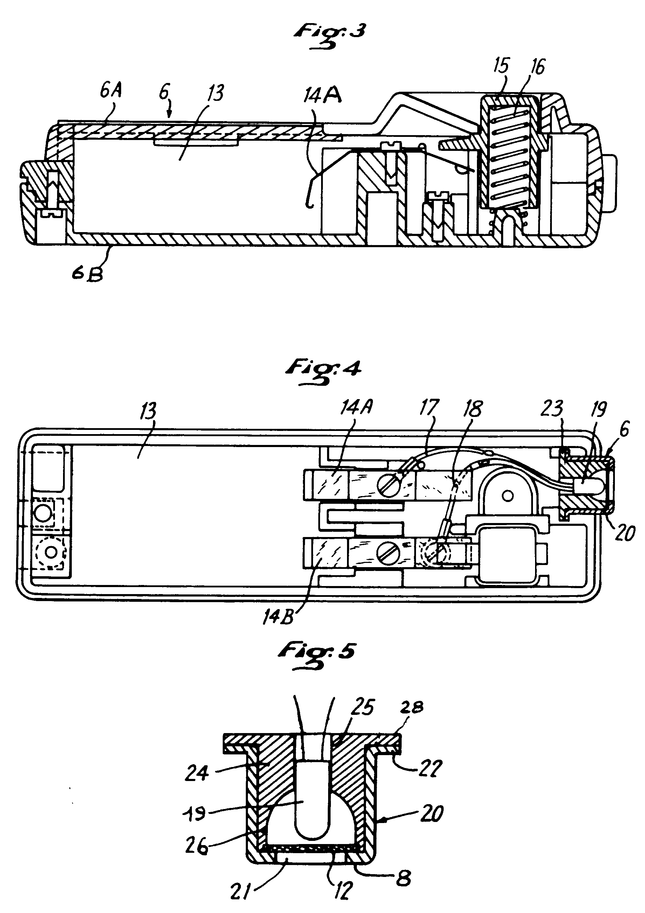

Dans les documents antérieurs analysés plus haut, l'élément chauffant est une ampoule électrique. Un élément chauffant de ce genre est disponible dans le commerce, fabriqué économiquement en grande série. On décrira donc maintenant en se reportant aux figures 3 à 5 un mode préféré de réalisation de l'invention utilisant une ampoule électrique. Mais, comme on le montrera, la détermination de l'ampoule convenable n'est pas aussi facile qu'il paraît.

La figure 3 montre que le corps creux allongé 6 de l'appareil est fabriqué en deux moitiés 6A , 6B qui sont assemblées l'une à l'autre dans un plan médian. Dans une première partie en sens longitudinal du volume du corps creux est prévu le logement 13 d'une source de courant électrique (pile ou accumulateur) avec deux lamelles flexibles 14A, 14B pour assurer le contact avec les bornes de cette source. Ces lamelles 14A, 14B s'étendent dans la deuxième partie en sens longitudinal du corps creux 6 dans laquelle se trouve un bouton 15, associé à un ressort de rappel 16, pour la commande de la fermeture et de l'ouverture du circuit qui est raccordé aux lamelles flexibles 14A, 14B. Ce circuit comprend, enplus du bouton poussoir 15, deux conducteurs 17, 18 qui aboutissent à une ampoule 19 (figure 4). Cette ampoule 19 est montée en retrait par rapport à une partie extrême ouverte 7 du corps 6.

La partie extrême 7 est saillante par rapport à une face extrême du corps 6. Elle est constituée par une coupelle 20 (mieux visible sur la figure 5) qui a à une extrémité une ouverture 21 d'un diamètre de 6 mm et à son extrémité opposée une collerette 22. Dans les deux moitiés6A, 6B du corps 6 sont prévus deux demi-évidements opposés avec des rainures 23 de sorte que la coupelle 20 peut être contenue en grande partie dans les demi-évidements avec sa collerette 22 engagée dans les rainures 23.

La coupelle 20 (figure 5) contient un réflecteur 24 qui est un corps plein ayant un trou central longitudinal 25 d'un diamètre de 3 mm pour contenir avec frottement une ampoule électrique cylindrique 19. Dans la partie du réflecteur 24 proche de l'ouverture 21 est creusé un évidement ouvert à surface sensiblement réfléchissante 26 dans lequel débouche le trou 25 de sorte que le filament de l'ampoule 19 est au foyer de cette surface 26 . A sa partie extrême opposée à la surface réfléchissante 26 le réflecteur 24 est pourvue aussi d'une collerette 28 qui s'applique contre la collerette 22 de la coupelle 20 quand le réflecteur 24 est à sa place dans la coupelle 20. Après montage de celle-ci dans le corps 6, les deux collerettes 22, 28 sont contenues dans les rainures 23 des deux moitiés 6A, 6B.

Il est possible, en plus, de prévoir comme représenté sur la figure 5 une grille 12 de protection serrée par la face extrême du réflecteur 24 contre la face intérieure de la paroi extrême dans laquelle est ménagée l'ouverture 21.

L'ampoule 19 est disponible dans le commerce; elle a une tension nominale d'alimentation de 5 V environ mais la source d'énergie à placer dans le logement 13 doit avoir une tension nominale de 9 V. L'ampoule est donc survoltée pendant l'utilisation et elle développe une puissance de 1, 25 W environ; comme elle a un diamètre de 3 mm, sa section droite est de 7 mm ² environ et elle fournit donc une puissance de 0,17 W environ par mm² de sa section droite (0,17 W/mm²). Cette section droite est dans un plan parallèle au plan de l'ouverture 21.

L'ampoule 19 se trouve dans le commerce sous les références OR 715 - 5 V 115 mA - T1 3,17 X 6,35; d'autres ampoules pourraient convenir mais il est important que la tension d'alimentation soit supérieure à 50 % au moins à la tension nominale. Dans le présent exemple, la tension d'alimentation (9V) est supérieure de 80 % à la tension nominale de l'ampoule. Bien entendu, plus la surtension est importante, plus la durée de vie de l'ampoule est réduite; une surtension de 100 % entraîne une destruction presque immédiate du filament de l'ampoule. Une surtension comprise entre 50 % et 90 %, de préférence 80 %, est la plus avantageuse.

Pendant l'utilisation de l'appareil décrit ci-dessus, la face extrême extérieure 8 de la coupelle 20 est appliquée contre la peau. La distance entre le plan de cette face extrême 8 et le point de l'ampoule 19 qui en est le plus proche est de 2 mm environ. Cette distance se règle et se modifie facilement (comme il peut être nécessaire quand la tension électrique fournie par la source d'alimentation commence à baisser) grâce au montage coulissant dur de l'ampoule 19 dans le réflecteur 24.

La grille 12 est constituée, par exemple, par une toile métallique à fil de laiton ayant un diamètre de 0,30 mm, tissé,ayant des mailles de 500 microns environ, soit un pourcentage de vide de 40 % environ.

Au moins la coupelle 20 et le réflecteur 24, et de préférence la totalité du corps allongé 6 sont réalisés en matière mauvaise conductrice de la chaleur.

Une éventuelle suppression du réflecteur 11 ou de la surface réfléchissante 26 aurait le désavantage que la puissance émise par l'ampoule en direction de la peau s'en trouve diminuée, de sorte que la durée d'échauffement de la peau en serait augmentée.

## Revendications

1. Appareil pour un chauffage localisé pour la destruction du venin injecté dans la peau par la piqûre de certains insectes et animaux marins, ayant un corps (6) contenant un élément thermo-électrique (9, 19) de chauffage, dans lequel le corps (6) est allongé et creux avec une extrémité ouverte (7), l'élément thermo-électrique (9, 19) monté dans ce corps creux est en retrait par rapport à cette extrémité ouverte (7), un réflecteur (11, 26) est placé dans le même corps en arrière dudit élément thermo-électrique par rapport à l'extrémité ouverte (7), une source de fourniture d'énergie est logée dans le corps et reliée de façon interruptible à volonté à l'élément thermo-électrique, et dans lequel le rapport entre la puissance de l'élément thermo-électrique de chauffage et la surface libre de l'extrémité ouverte est compris entre 0,03 W/mm² et 0,30 W/mm², de préférence entre 0,04 et 0,25 W/mm².

2. Appareil selon la revendication 1 caractérisé en ce que la source de flux calorifique a une puissance comprise entre 0,8 W et 3 W.

3. Appareil selon la revendication 1 dans lequel la source de flux électrique est une ampoule électrique, caractérisé en ce que cette ampoule développe une puissance comprise entre 0,12 W/mm² et 0,20 W/mm², la surface étant celle de la section de cette ampoule par un plan parallèle au plan (8) contenant l'extrémité ouverte (7) du corps (6) de l'appareil, et la distance entre ce plan contenant l'extrémité ouverte et le point de l'ampoule le plus proche de ce même plan étant de 2 mm environ.

4. Appareil selon la revendication 3 caractérisé en ce que ladite puissance développée par l'ampoule est de 0,17 W/mm² environ.

5. Appareil selon la revendication 4 caractérisé en ce que la surface libre de l'extrémité ouverte du corps est de 28 mm² environ.

6. Appareil selon la revendication 1 caractérisé en ce que la source de flux calorifique est une ampoule électrique ayant une puissance de 1,25 W environ et un diamètre extérieur de 3 mm environ dans un plan parallèle au plan contenant l'extrémité ouverte du corps.

7. Appareil selon la revendication 6 caractérisé en ce que la surface libre de l'extrémité ouverte (7) du corps (6) a une valeur de 28 mm² environ et elle est située à une distance de 2 mm environ du point de l'ampoule le plus proche.

8. Appareil selon la revendication 3, caractérisé en ce que l'ampoule est alimentée par une souce d'énergie électrique ayant une tension supérieure de 50 % à 90 % à la tension nominale de cette ampoule.

9. Appareil selon la revendication 8 caractérisé en ce que l'ampoule électrique (19) à une tension nominale de 5 V environ et la source de fourniture d'énergie est une pile électrique ou un accumulateur électrique ayant une tension nominale de 9 V environ.

10. Appareil selon la revendication 8 caractérisé en ce que l'ampoule (19) est cylindrique et qu'elle est introduite à frottement dur dans un trou (25) ménagé dans le corps plein d'un réflecteur (26) ayant un évidement ouvert,à surface réfléchissante (26), dans lequel débouche le trou (25).

## Claims

1. Apparatus for localized heating for destroying venom injected into the skin by the sting of certain insects and sea creatures, having a body (6) containing a thermo-electric heating element (9,19), wherein the body (6) is elongated and hollow with an open end (7), the thermo-electric heating element (9,19) mounted in said hollow body is set back from said open end (7), a reflector (11,26) is placed in said body behind said thermo-electric element relative to the open end (7), an energy supply source is lodged in the body and connected to the thermo-electric element in a manner which is interruptible at will, and wherein the ratio between the power of the thermo-electric heating element and the free area of the open end lies in the range 0.03 W/mm² and 0.30 W/mm², preferably in the range 0.04 W/mm' and 0.25 W/mm².

2. Apparatus according to claim 1, characterized in that the heat flux source has a power lying in the range 0.8 W and 3 W.

3. Apparatus according to claim 1, wherein the heat flux source is an electric lightbulb, characterized in that said bulb develops a power lying in the range 0.12 W/mm² to 0.20 W/mm2, with the area being the area of the cross-section of said bulb on a plane parallel to the plane (8) containing the open end (7) of the body (6) of the apparatus, and the distance between said plane containing the open end and the point of the bulb closest to said plane being about 2mm.

4. Apparatus according to claim 3, characterized in that said power developed by the bulb is about 0.17 W/mm².

5. Apparatus according to claim 4, characterized in that the free area of the open end of the body is about 28 mm².

6. Apparatus according to claim 1, characterized in that the source of heat flux is an electric bulb having a power of about 1.25 W and an outside diameter in a plane parallel to the plane containing the open end of the body of about 3 mm.

7. Apparatus according to claim 6, characterized in that the free area of the open end (7) of the body (6) is about 28 mm² and is situated at a distance of about 2 mm from the closest point of the bulb.

8. Apparatus according to claim 3, characterized in that the bulb is fed from an electric energy source having a voltage which is 50% to 90% greater than the nominal voltage of said bulb.

9. Apparatus according to claim 8, characterized in that the electric bulb (19) has a nominal voltage of about 5 V and the energy supply source is an electric battery or rechargeable battery having a nominal voltage of about 9 V.

10. Apparatus according to claim 8, characterized in that the bulb (19) is cylindrical and in that it is a push fit into a hole (25) provided in the solid body of a reflector (26) having an open recess with a reflecting surface (26) into which the hole (25) opens out.

## Patentansprüche

1. Gerät zur lokalen Erwägung zur Zerstörung des Giftes, das durch den Stich bestimmter Insekten und Meereslebewesen in die Haut eingespritzt wurde, mit einem ein thermoelektrisches Heizelement (9, 19) enthaltenden Gehäuse (6), in welchem Gerät das Gehäuse (6) länglich und hohl mit einem offenen Ende (7) ist, das in diesem hohlen Gehäuse (6) montierte thermoelektrische Element (9, 19) gegenüber diesem offenem Ende (7) zurückversetzt ist, ein Reflektor (11, 26) in dem selben Gehäuse bezüglich des offenen Endes (7) hinter dem genannten thermoelektrischen Element angeordnet ist, eine Energieversorgungsquelle in dem Gehäuse angeordnet und in willkürlich unterbrechbarer Weise mit dem thermoelektrischen Element verbunden ist, und in welchem das Verhältnis zwischen der Leistung des thermoelektrischen Heizelementes und der freien Fläche des offenen Endes zwischen 0,03 W/mm² und 0,3 W/mm², vorzugsweise zwischen 0,04 und 0,25 W/mm² beträgt.

2. Gerät nach Anspruch 1, dadurch **gekennzeichnet,** daß die Wärmflußquelle eine Leistung zwischen 0,8 W und 3 W hat.

3. Gerät nach Anspruch 1, in dem die Quelle des elektrischen Flusses eine elektrische Glühbirne ist, dadurch **gekennzeichnet,** daß diese Glühbirne eine Leistung zwischen 0,12 W/mm² und 0,20 W/mm² entwickelt, wobei die Fläche gleich der Schnittfläche durch diese Glühbirne ist, die von einer zu der das offene Ende (7) des Gehäuses (6) des Gerätes enthaltenden Ebene (8) parallel ist, wobei der Abstand zwischen dieser das offene Ende enthaltenden Ebene und dem dieser selben Ebene nächst gelegenen Punkt der Glühbirne ungefähr 2mm beträgt.

4. Gerät nach Anspruch 3, dadurch **gekennzeichnet,** daß die genannte von der Glühbirne entwickelte Leistung ungefähr der 0,7 W/mm² beträgt.

5. Gerät nach Anspruch 4, dadurch **gekennzeichnet,** daß die freie Fläche des offenen Endes des Gehäuses ungefähr 28 mm² beträgt.

6. Gerät nach Anspruch 1, dadurch **gekennzeichnet,** daß die Wärmeflußquelle eine elektrische Glühbirne ist, die eine Leistung von ungefähr 1,25 W hat und einen Außendurchmesser von ungefähr 3 mm in einer Ebene hat, die parallel zur der das offene Ende des Gehäuses enthaltende Ebene ist.

7. Gerät nach Anspruch 6, dadurch **gekennzeichnet,** daß die freie Fläche des offenen Endes (7) des Gehäuses (6) einen Wert von ungefähr 28 mm² hat und daß sie in einem Abstand von ungefähr 2 mm von dem nächst gelegenen Punkt der Glühbirne liegt.

8. Gerät nach Anspruch 3, dadurch **gekennzeichnet,** daß die Glühbirne von einer elektrischen Energiequelle mit einer Spannung gespeist wird, die 50 % bis 90 % über der Nennspannung der Glühbirne liegt.

9. Gerät nach Anspruch 8, dadurch **gekennzeichnet,** daß die elektrische Glühbirne (19) eine Nennspannung von ungefähr 5 V hat und daß die Energieversorgungsquelle eine elektrische Batterie oder ein elektrischer Akkumulator mit einer Nennspannung von ungefähr 9 V ist.

10. Gerät nach Anspruch 8, dadurch **gekennzeichnet,** daß die Glühbirne (19) zylindrisch ist und daß sie mit festem Reibschluß in ein Loch (25) eingesetzt ist, das in dem vollem Material eines Reflektors (26) ausgebildet ist, der eine offene Aussparung mit reflektierender Oberfläche (26) hat, in welche das Loch (25) mündet.
